# EUROPEAN PATENT SPECIFICATION

(11) **EP 1 552 834 B1**
(45) Date of publication and mention of the grant of the patent: **02.05.2007**
(21) Application number: 03754096.0
(22) Date of filing: 10.10.2003
(51) Int. Cl.: A61K 31/445, A61P 9/00, A61P 9/10, A61P 21/04, A61P 25/00, A61P 25/14, A61P 25/16, A61P 25/28, A61P 25/02, A61P 43/00

(54) **N-ACETYL-L-PIPECOLIC ACID AS NEUROTROPHIC FACTOR PRODUCTION PROMOTER**
N-ACETYL-L-PIPECOLIC SÄURE ALS PROMOTER DER PRODUKTION DES NEUROTROPHISCHEN FAKTORS
ACIDE N-ACETYL-L-PIPECOLIQUE COMME PROMOTEUR DE LA PRODUCTION DU FACTEUR NEUROTROPHIQUE

(30) Priority: 15.10.2002 JP 2002300247
(43) Date of publication of application: 13.07.2005
(73) Proprietor: Astellas Pharma Inc., Chuo-ku, Tokyo (JP)
(72) Inventor: FURUKAWA, Shoei, Gifu 502-0003 (JP); NITTA, Atsumi, Nagoya-shi, Aichi 460-0007 (JP)
(74) Representative: Gille Hrabal Struck Neidlein Prop Roos
(86) International application number: PCT/JP2003/013099
(87) International publication number: WO 2004/035053

(56) References cited:
- WO-A1-97/16190
- US-A- 5 696 135
- US-A- 5 798 355
- HAMILTON G S ET AL: "NEUROIMMUNOPHILIN LIGANDS AS NOVEL THERAPEUTICS FOR THE TREATMENT OF DEGENERATIVE DISORDERS OF THE NERVOUS SYSTEM" CURRENT PHARMACEUTICAL DESIGN, BENTHAM SCIENCE PUBLISHERS, SCHIPHOL, NL, vol. 3, no. 4, 1997, pages 405-428, XP000980342 ISSN: 1381-6128
- SMITH, B. AMOS: 'TOTAL SYNTHESIS OF RAPAMYCIN AND DEMETHOXYRAPAMYCIN' J.AM.CHEM.SOC. vol. 117, no. 19, 1995, pages 5407 - 5408, XP002079789
- ERIC J. TOONE ET AL.: 'Enzyme in organic synthesis.40.Evaluation of the enantioselectivity of the pig liver esterase catalyzed hydrolyses of racemic piperidine carboxylic acid esters' CAN.J.CHEM. vol. 65, no. 12, 1987, pages 2722 - 2726, XP002974913

## Description

The present invention relates to N-acetyl-L-pipecolic acid as a novel neurotrophic factor production promoter useful for the prophylaxis or treatment of neurodegenerative diseases and the like.

### Background Art

The central and peripheral nervous systems include a number of nerve cells that transmit neuronal information. However, since nerve cells cease division around birth, once they are damaged, their regeneration is difficult. As a result, the same nerve cells bear the function throughout the entire life, and therefore, various neurotrophic factors are considered to play a large part in the differentiation, elongation of axon, synapse formation and survival and functional maintenance thereof. Of these, neurotrophin family is a representative family that has been comparatively well studied. The family has been reported to include nerve growth factor (hereinafter to be referred to as "NGF"), brain-derived neurotrophic factor (hereinafter to be referred to as "BDNF"), neurotrophin-3 (hereinafter to be referred to as "NT-3"), NT-4/5, NT-6 and the like. Besides neurotrophin, glia cell derived neurotrophic factor (hereinafter to be referred to as "GDNF") has been recently identified and considered to play an important role as a neurotrophic factor. However, the detail thereof has not been elucidated.

Meanwhile, along with the aging of the society, the number of patients with neurodegenerative diseases, such as Alzheimer's disease and Parkinson's disease, is increasing. These diseases are intractable and progressive, and a basic treatment method thereof has not been established. Under the circumstances, application of neurotrophic factor to the treatment of neurodegenerative diseases has been considered. However, since neurotrophic factor is a protein, which is susceptible to degradation in blood and cannot pass the blood-brain barrier, an effect in the brain cannot be expected by peripheral administration. Furthermore, direct infusion into the brain is ethically and technically limited. Thus, once a compound permitting administration from the periphery, which is capable of increasing the expression of these factors in the brain, can be found, the possibility arises that it can be actively used as a therapeutic drug (see e.g. WO99/62879 (*tokuhyo* 2002-516903) or WO99/62881 (*tokuhyo* 2002-516905)).

A recent report has documented that immunosuppressants inhibit brain ischemia and traumatic encephalopathy. It has also been reported that immunophilin, which is an intracellular binding protein of immunosuppressants, is abundantly present not only in the immune systems but also in the central nervous system and mediates a central nerve protecting effect on nerve cells. However, immunosuppressive action is disadvantageous for the application of immunophilin ligand aiming at neuroprotection. Therefore, creation of a neuroprotective substance free of an immunosuppressive action has been desired.

Inhibitors of rotamase activity are described in US -A- 5,696,135 and WO 97 16190. Neurotrophic pipecolic acid derivatives with an affinity for FKBP-type immunophilins are described as a suitable treatment for neurological disorders. Hamilton & Steiner (Curr. Pharm. Design, 1997; 3: 405-42) have described the use of small molecule ligands which bind to FKBP 12 as effective agents in experimental animal models of neurodegenerative disease without displaying at the same an immunosuppressive action.

### Disclosure of the Invention

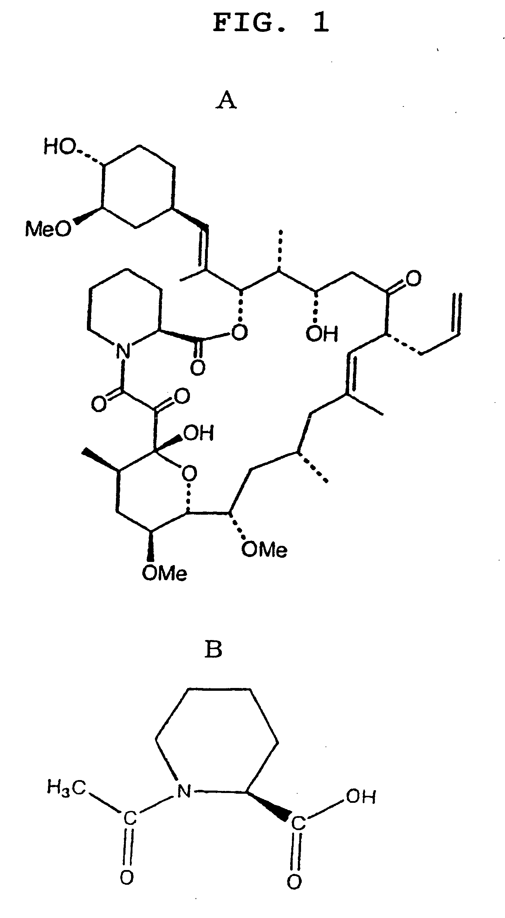

The present inventors took note of the structure wherein tacrolimus (Fig. 1-A), which is an immunosuppressant, interacts with its binding protein, and investigated the neuroprotective action of N-acetyl-L-pipecolic acid (Fig. 1-B, hereinafter to be referred to as "APA") obtained by stabilizing the chemical structure of a part thereof, and found that APA has a superior neurotrophic factor production promoting effect, which resulted in the completion of the present invention.

Accordingly, the present invention is characterized by the following.
(1) A neurotrophic factor production promoter comprising N-acetyl-L-pipecolic acid or a pharmacologically acceptable salt thereof as an active ingredient.
(2) The promoter of (1), which is administered to a human or an animal for the prophylaxis or treatment of a neurodegenerative disease.
(3) The promoter of (2), wherein the neurodegenerative disease is Alzheimer's disease, Parkinson's disease, spinal injury, Huntington's disease, cerebral infarction, head trauma, multiple sclerosis, amyotrophic lateral sclerosis, or diabetic or drug-induced peripheral neuropathy or retinal neuropathy.
(4) The promoter of any of (1) to (3), wherein the neurotrophic factor is neurotrophin.
(5) Use of N-acetyl-L-pipecolic acid or a pharmaceutically acceptable salt thereof for the production of an agent for the prophylaxis or treatment of a neurodegenerative disease.
(6) Use of N-acetyl-L-pipecolic acid or a pharmaceutically acceptable salt thereof for the production of a neurotrophic factor production promoter.

As used herein, the "neurotrophic factor" is a generic term of proteins having a physiological action such as survival and maintenance of nerve cells, promotion of neuronal differentiation and the like, like NGF, as mentioned above, and specifically refers to a neurotrophin family including NGF, BDNF, NT-3, NT-4/5 and NT-6, and further, GDNF, glia growth factor (GGF2), central nervous system growth factor (AF-1) and the like.

By the "neurotrophic factor production promoter" is meant a pharmaceutical agent that shows, upon in vivo or in vitro contact with nerve cells, an action of inducing or promoting the production (synthesis) of a neurotrophic factor from the cell.

A "neurotrophin" in living organisms means a neurotrophic factor secreted from a target cell of neuronal growth or a cell extending toward a target, or a neurotrophic factor that helps nerves (neuron) grow, differentiate and survive by autocrine or paracrine to form neural circuits (synapse), and NGF, BDNF, NT-3, NT-4/5 and NT-6 are specifically known at present, as mentioned above. It means a protein group having similar structures and highly homologous amino acid sequences.

The "neurodegenerative disease" is a generic term of diseases associated with drop out and necrosis of nerve cells in the central or peripheral nervous system. Representative examples thereof include Alzheimer's disease, Parkinson's disease, spinal injury, Huntington's disease, cerebral infarction, head trauma, multiple sclerosis and amyotrophic lateral sclerosis. In addition, diabetic or drug-induced peripheral neuropathy and retinal neuropathy are also included in the concept of neurodegenerative diseases in the present invention.

A "pharmaceutically acceptable salt" is a conventional non-toxic salt, which specifically includes metal salts such as alkali metal salts (e.g., sodium salt and potassium salt) and alkaline earth metal salts (e.g., calcium salt and magnesium salt), inorganic acid addition salts (e.g., hydrochloride, hydrobromide, sulfate, phosphate etc.), organic carboxylic acid or sulfonic acid adducts (e.g., formate, acetate, trifluoroacetate, maleate, tartrate, fumarate, methanesulfonate, benzenesulfonate, toluenesulfonate etc.), and salts with basic or acidic amino acids (e.g., arginine, aspartic acid, glutamic acid etc.).

Now, the formulation of preparations and the dose of the neurotrophic factor production promoter of the present invention are explained. The neurotrophic factor production promoter of the present invention can be administered orally, parenterally (including intravenous, intraperitoneal, subcutaneous and intramuscular injections) or externally (topically) (including rectal, transdermal, instillation and transnasal administrations) in the form of a conventional pharmaceutical preparation such as capsule, microcapsule, tablet, granule, powder, troche, pill, ointment, suppository, injection, suspension, syrup, emulsion, liquid, enteric coated agent, spray, inhalant, eye drop, nose drop and the like.

The above-mentioned pharmaceutical preparations can be produced according to conventional methods using various organic or inorganic carriers conventionally used for formulation of preparations, such as excipients (e.g., sucrose, starch, mannit, sorbit, lactose, glucose, cellulose, talc, calcium phosphate, calcium carbonate etc.), binders (e.g., cellulose, methyl cellulose, hydroxymethyl cellulose, polypropylpyrrolidone, gelatin, gum arabic, polyethylene glycol, sucrose, starch etc.), disintegrants (e.g., starch, carboxymethyl cellulose, hydroxypropylstarch, sodium hydrogen carbonate, calcium phosphate, calcium citrate etc.), lubricants (e.g., magnesium stearate, aerosyl, talc, sodium lauryl sulfate etc.), corrigents (e.g., citric acid, menthol, glycine, orange powder etc.), preservatives (e.g., sodium benzoate, sodium bisulfite, methylparaben, propylparaben etc.), stabilizers (e.g., citric acid, sodium citrate, acetic acid etc.), suspending agents (e.g., methyl cellulose, polyvinylpyrrolidone, aluminum stearate etc.), dispersing agents (e.g., hydroxypropylmethyl cellulose etc.), diluents (e.g., water etc.), base waxes (e.g., cacao butter, white petrolatum, polyethylene glycol etc.) and the like.

The dose of the neurotrophic factor production promoter of the present invention only needs to be an amount sufficient to provide a desired therapeutic (or prophylactic) effect, which is, for example, 0.01 mg/kg - 100 mg/kg, preferably 0.1 mg/kg - 10 mg/kg, for oral or parenteral administration. The neurotrophic factor production promoter of the present invention can be generally administered in a unit dose of 0.1 mg/individual - 1000 mg/individual, preferably 5 mg/individual - 500 mg/individual, 1 to 4 times a day. However, the above-mentioned dose may be appropriately increased or decreased depending on the age and body weight of patients, symptom or administration method.

### Examples

The present invention is explained in more detail by referring to Experimental Examples and Examples.

### Experimental Example 1 (Effect of APA on the expression of neurotrophic factor in the brain of normal mouse)

Test method: 5-week-old male ddy mice (Japan SLC, Inc.) were divided into the following three groups (9 mice per group) and subjected to a test.
Group 1 (control) Phosphate buffered saline (PBS) was intraperitoneally administered to the mice once a day for 7 days.
Group 2 APA (0.75 mg/kg) dissolved in PBS was intraperitoneally administered to the mice once a day for 7 days.
Group 3 APA (7.5 mg/kg) dissolved in PBS was intraperitoneally administered to the mice once a day for 7 days.

In every group, the mice were decapitated at 24 hr after the final administration and the cerebral cortex and striatum thereof were removed.

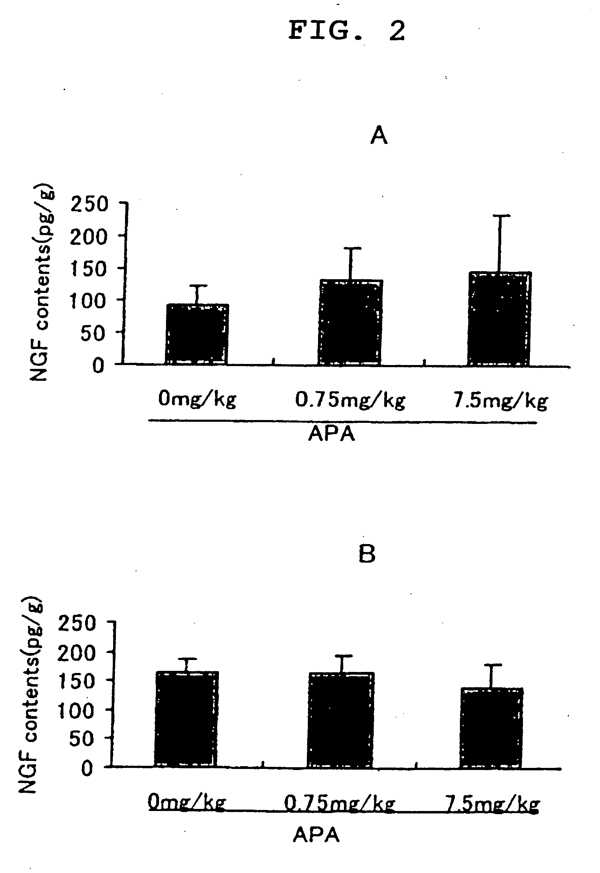

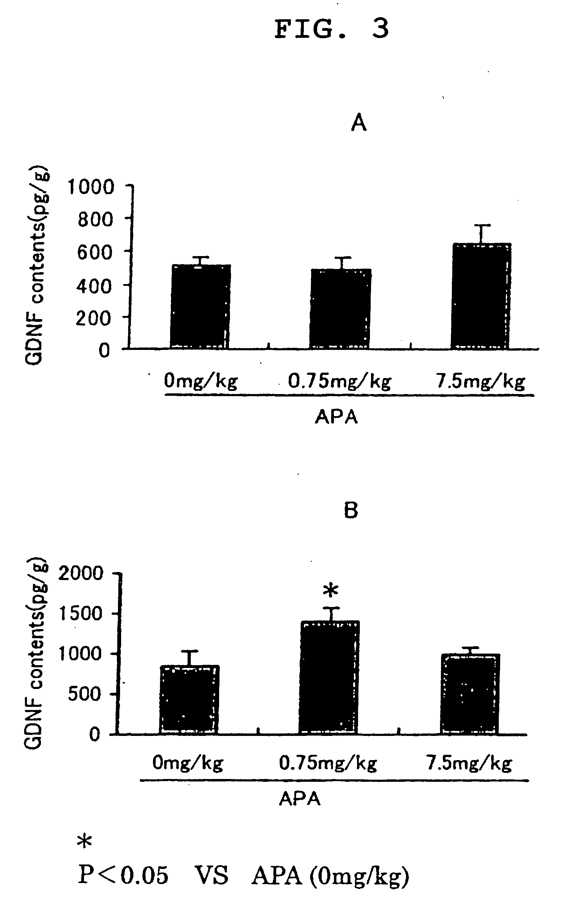

Preparation of samples for enzyme assay (EIA): The removed cerebral cortex and striatum were weighed, a homogenizing buffer (0.1M Tris-phosphate buffer (pH 7.4) containing 1M sodium chloride, 2% BSA, 2mM EDTA, 0.2% sodium azide) supplemented with aprotinin (80 TIU/L) was added in an amount of 19-fold volume of the weight, and the mixture was sonicated 30 times. After centrifugation (100,000 g x 30 min) at 4°C, the supernatant was separated and the same amount of chloroform was added. The mixture was thoroughly stirred and centrifuged (20,000 g x 10 min). The supernatant was separated and used as a sample for EIA (cryopreserved at -20°C until use).
Measurement of neurotrophic factor by EIA:
The concentration of neurotrophic factor in each sample was measured using EIA according to the method of Furukawa et al. (Journal of Neurochemistry), 40, 734-744 (1983)} in the case of NGF, and using EIA according to the method of Nitta et al. {pp. 463-467 of "Mapping the progress of Alzheimer's and Parkinson's Disease" (2002) edited by Mizuno et al.}, in the case of GDNF. The obtained values were multiplied by 20 to give a neurotrophic factor content of each brain region.
Test results: The mice that underwent intraperitoneal administration of APA for 7 consecutive days did not show abnormal behavior, alopecia or decrease of body weight, as compared to control mice. The NGF expression amount in the striatum did not show remarkable difference from the control, due to the APA administration (Fig. 2-B), but that in the cerebral cortex tended to increase in a dose-dependent manner due to the APA administration (Fig. 2-A). As for GDNF expression amount, no remarkable difference was found in the cerebral cortex as compared to the control, due to the APA administration (Fig. 3-A), but in the striatum, a 66.0% increase was observed as compared to the control, due to the APA (0.75 mg/kg) administration (Fig. 3-B).

### Experimental Example 2 (Neuroprotective effect of APA via promotion of neurotrophic factor production in mouse model of Parkinson's disease)

Test method: 5-week-old male ddy mice (Japan SLC, Inc.) were divided into the following four groups (9 mice per group) and subjected to a test. Under pentobarbital anesthesia and without craniotomy, a needle of a microsyringe was punctured through the head skin into the striatum of the right brain, and 1 µL of 6-hydroxydopamine (6-OHDA, Sigma Co., 11.5 g/µL) dissolved in physiological saline containing 0.05% ascorbic acid was injected.
Group 1 (control) Physiological saline containing only ascorbic acid was injected, whereby the striatum was not damaged. Thereafter, PBS alone was intraperitoneally administered to the mice once a day for 7 days.
Group 2 PBS was intraperitoneally administered to the mice once a day for 7 days starting from the day the striatum was destroyed by 6-OHDA.
Group 3 APA (0.75 mg/kg) dissolved in PBS was intraperitoneally administered to the mice once a day for 7 days starting from the day the striatum was destroyed by 6-OHDA.
Group 4 APA (7.5 mg/kg) dissolved in PBS was intraperitoneally administered to the mice once a day for 7 days starting from the day the striatum was destroyed by 6-OHDA.

In every group, the mice were decapitated at 24 hr after the final administration and the cerebral cortex was removed.

Functional evaluation of dopaminergic nerve: The mouse models of Parkinson's disease of each of the above-mentioned groups were allowed to search freely in a transparent cylindrical container (glass, diameter 9 cm - height 12 cm) for 10 min, methamphetamine (DAINIPPON Pharmaceutical Co., Ltd., 10 mg/kg) was intraperitoneally administered, and the number of cycling in 10-20 min after administration was counted. In this test, cycling movement (one side cycling movement) in the right direction was observed, because the right striatum had been destroyed.

### Preparation of samples for EIA and measurement of neurotrophic factor by EIA: Both were performed in the same manner as in Experimental Example 1.

Results: In the functional evaluation of dopaminergic nerve, the group with destruction of the striatum and free of APA administration showed 86.0±14.1 times of one side cycling movement. In contrast, when 0.75 or 7.5 mg/kg of APA was administered for 7 days after destruction of the striatum, the one side cycling movement was suppressed to 40.9±17.3 times and 47.1±13.1 times, respectively (Fig. 4). The NGF expression amount in the cerebral cortex decreased to 50.0% upon injection of 6-OHDA, but upon administration of APA (0.75 and 7.5 mg/kg), an increase of 11.2% and 30.7% was observed, respectively (Fig. 5). In addition, the GDNF expression amount in the cerebral cortex increased by 74.9% and 232.4%, respectively, by the administration of 0.75 and 7.5 mg/kg of APA (Fig. 6).

### Experimental Example 3 (effect of APA on walking ability of rats with spinal injury)

Test method: 7-week-old male Wistar rats (Japan SLC, Inc.) were divided into the following three groups (12-17 rats per group) and subjected to a test. Pentobarbital sodium (DAINIPPON Pharmaceutical Co., Ltd., 35 mg/kg) was intraperitoneally injected to the rats, median incision was applied to the back, and paraspinal muscle was bluntly detached. After laminectomy of the 12th thoracic vertebra, the left spinal cord was cut at the same position with a sharp cutting tool. After 5 hr from the cutting, the rats confirmed to have anesthetic disorder in the left hind paw were subjected to the following test.
Group 1 PBS was intraperitoneally administered to rats with uninjured spinal cord (sutured without cutting the spinal cord after laminectomy of the 12th thoracic vertebra).
Group 2 PBS was intraperitoneally administered to rats with injured spinal cord.
Group 3 APA (0.75 mg/kg) dissolved in PBS was intraperitoneally administered to rats with injured spinal cord.

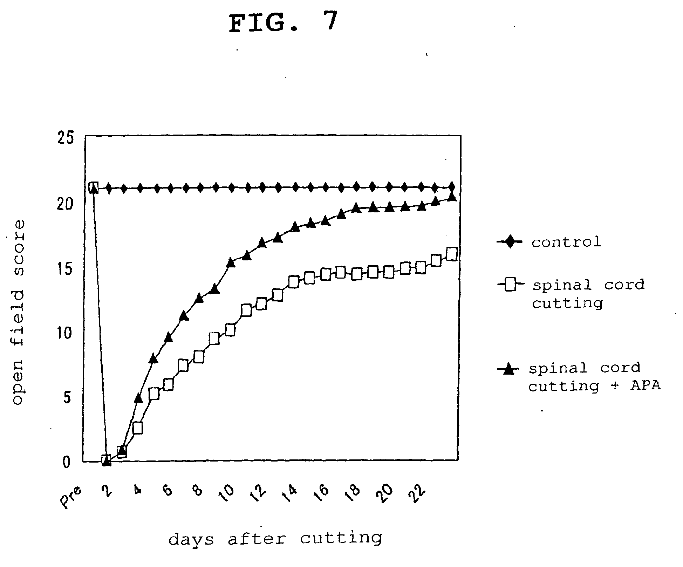

In every group, the administration was performed once a day for 20 days from the day of spinal injury. Evaluation method of walking ability of rats with injured spinal cord: The rats were placed in a box (length 1 m x width 1 m x height 30 cm) painted in gray, allowed to search freely, and evaluated in 21 levels according to 21-Point Basso-Besttie-Bresnahan Locomotor Rating Scale (BBB scale).
Test results: The BBB scale decreased to 0 immediately after spinal injury. The effect of APA administration was not observed for the first 2 days, but the level of walking ability showed improvement from day 3 by APA administration. In the APA administration group, the level showed recovery to almost the same level as before injury at day 23. The level in the rats of the spinal injury group gradually rose with the lapse of time, and reached score 15 at day 16, though with no recovery beyond this level (Fig. 7).

A formulation example of the present invention is shown below.

### Example 1 (capsule)

### APA 5 mg, lactose 80 mg

The above-mentioned components are mixed and filled in a regular hard gelatin capsule to give a capsule.

### Brief Description of the Drawings

Fig. 1(A) shows the structural formula of tacrolimus and Fig. 1(B) shows the structural formula of N-acetyl-L-pipecolic acid.
Fig. 2(A) shows the effect of APA on the NGF expression in the cerebral cortex of mouse and Fig. 2(B) shows the effect of APA on the NGF expression in the striatum of mouse.
Fig. 3(A) shows the effect of APA on the GDNF expression in the cerebral cortex of mouse and Fig. 3(B) shows the effect of APA on the GDNF expression in the striatum of mouse.

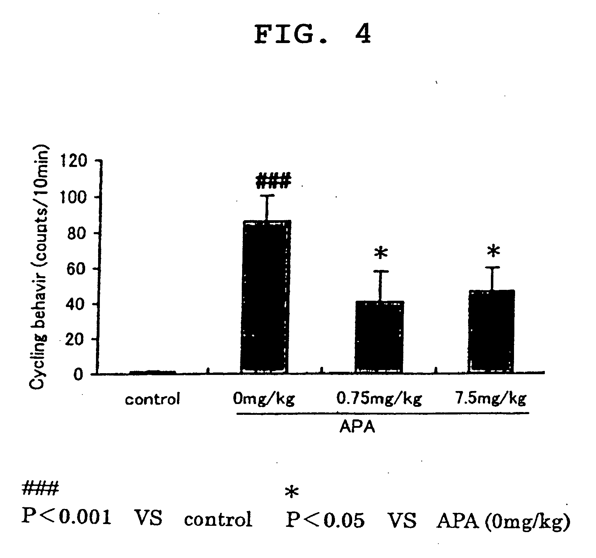

Fig. 4 shows a one side cycling movement-suppressive effect of APA on the function of dopaminergic nerve in the mouse model of Parkinson's disease.

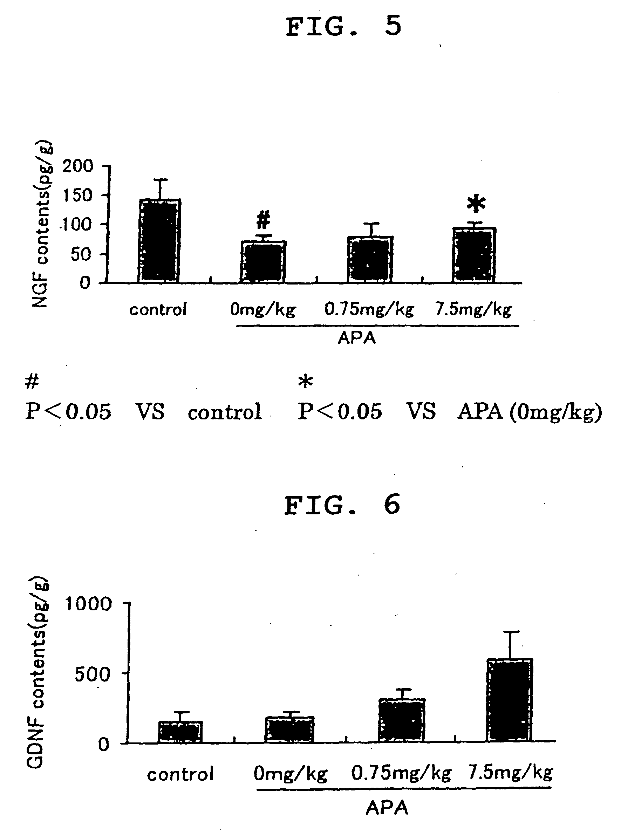

Fig. 5 shows the effect of APA on the NGF expression in the cerebral cortex of the mouse model of Parkinson's disease.
Fig. 6 shows the effect of APA on the GDNF expression in the cerebral cortex of the mouse model of Parkinson's disease.
Fig. 7 shows the effect of APA on the improvement of the walking ability of rats with spinal injury.

### Industrial Applicability

According to the present invention, a safe pharmaceutical agent which induces and promotes biosynthesis of neurotrophic factors such as NGF, GDNF and the like, and which is effective for neurodegenerative diseases such as Alzheimer's disease, Parkinson's disease, spinal injury and the like can be provided.

## Claims

1. A neurotrophic factor production promoter comprising N-acetyl-L-pipecolic acid or a pharmacologically acceptable salt thereof as an active ingredient.

2. The promoter of claim 1, which is administered to a human or an animal for the prophylaxis or treatment of a neurodegenerative disease.

3. The promoter of claim 2, wherein the neurodegenerative disease is Alzheimer's disease, Parkinson's disease, spinal injury, Huntington's disease, cerebral infarction, head trauma, multiple sclerosis, amyotrophic lateral sclerosis, or diabetic or drug-induced peripheral neuropathy or retinal neuropathy.

4. The promoter of any of claims 1 to 3, wherein the neurotrophic factor is neurotrophin.

5. Use of N-acetyl-L-pipecolic acid or a pharmaceutically acceptable salt thereof for the production of an agent for the prophylaxis or treatment of a neurodegenerative disease.

6. Use of N-acetyl-L-pipecolic acid or a pharmaceutically acceptable salt thereof for the production of a neurotrophic factor production promoter.

## Patentansprüche

1. Promotor der Bildung des neurotrophen Faktors, umfassend N-Acetyl-L-pipecolinsäure oder ein pharmakologisch verträgliches Salz davon als aktiven Bestandteil.

2. Promotor nach Anspruch 1, welcher einem Menschen oder einem Tier zur Prophylaxe oder Behandlung von neurodegenerativen Erkrankungen gegeben wird.

3. Promotor nach Anspruch 2, wobei die neurodegenerative Erkrankung die Alzheimer-Erkrankung, Parkinson-Krankheit, spinale Verletzungen, Chorea Huntington, Hirninfarkt, Schädeltrauma, multiple Sklerose, amyotrophe Lateralsklerose oder durch Diabetes oder Drogen induzierte periphere Neuropathie oder retinale Neuropathie ist.

4. Promotor nach einem der Ansprüche 1 bis 3, wobei der neurotrophe Faktor Neurotrophin ist.

5. Verwendung von N-Acetyl-L-pipecolinsäure oder eines pharmazeutisch verträglichen Salzes davon zur Herstellung eines Mittels zur Prophylaxe oder Behandlung einer neurodegenerativen Erkrankung.

6. Verwendung von N-Acetyl-L-pipecolinsäure oder eines pharmazeutisch verträglichen Salzes davon zur Herstellung eines Promotors der Bildung des neurotrophen Faktors.

## Revendications

1. Promoteur de production de facteur neurotrophique comprenant de l'acide N-acétyl-L-pipécolique ou un sel pharmacologiquement acceptable de celui-ci en tant qu'un ingrédient actif.

2. Promoteur selon la revendication 1, qui est administré à un humain ou à un animal pour la prophylaxie ou le traitement d'une maladie neurodégénérative.

3. Promoteur selon la revendication 2, dans lequel la maladie neurodégénérative est la maladie d'Alzheimer, la maladie de Parkinson, une lésion de la moelle épinière, la chorée de Huntington, un infarctus cérébral, un traumatisme crânien, la sclérose en plaques, la sclérose latérale amyotrophique, ou une neuropathie rétinienne ou une neuropathie périphérique diabétique ou médicamenteuse.

4. Promoteur selon l'une quelconque des revendications 1 à 3, dans lequel le facteur neurotrophique est la neurotrophine.

5. Utilisation d'acide N-acétyl-L-pipécolique ou d'un sel pharmaceutiquement acceptable de celui-ci pour la production d'un agent destiné à la prophylaxie ou au traitement d'une maladie neurodégénérative.

6. Utilisation d'acide N-acétyl-L-pipécolique ou d'un sel pharmaceutiquement acceptable de celui-ci pour la production d'un promoteur de production de facteur neurotrophique.
